# EUROPÄISCHE PATENTANMELDUNG

(11) **EP 2 053 877 A1**
(43) Veröffentlichungstag der Anmeldung: **29.04.2009**
(21) Anmeldenummer: 07020434.2
(22) Anmeldetag: 18.10.2007
(51) Int. Cl.: H04R 25/00, A61B 5/12

(54) **Verfahren und Vorrichtung zur Anpassung eines Hörgeräts mittels DPOAE**

(71) Anmelder: Siemens Medical Instruments Pte. Ltd., Singapore 139959 (SG)
(72) Erfinder: Röhrlein, Gerhard, Dr., 91315 Höchstadt (DE)
(74) Vertreter: Maier, Daniel Oliver

(57) **Zusammenfassung**

Die Anpassung eines Hörgeräts (10) an einen Hörgerätsträger soll verbessert werden, wobei insbesondere auf die Mitwirkung des Hörgeräteträgers verzichtet werden soll, um Fehlanpassungen zu vermeiden. Hierzu wird ein Verfahren und eine Anpassvorrichtung vorgeschlagen, bei denen otoakustische Emissionen (14) gemessen, aus den Messungen eine Hörschwelle und/oder Unbehaglichkeitsschwelle geschätzt und anhand der geschätzten Hörschwelle und/oder Unbehaglichkeitsschwelle das Hörgerät (10) eingestellt wird. Das Gehör des Hörgerätsträgers wird gleichzeitig mit mindestens zwei Testsignalen (12) unterschiedlicher Frequenz (f1, f2) stimuliert. Die otoakustischen Emissionen (14) enthalten Verzerrungsprodukte (f3), so genannte "Distortion Products", die zum Schätzen der Hörschwelle herangezogen werden. Die "Distortion Product" OAE (DPOAE) kann sehr zuverlässig zur Bestimmung der Hörschwelle eingesetzt werden.

## Beschreibung

Die vorliegende Erfindung betrifft ein Verfahren zur Anpassung eines Hörgeräts durch Messen von otoakustischen Emissionen, Schätzen einer Hörschwelle und/oder einer Unbehaglichkeitsschwelle aus den Messungen und Einstellen des Hörgeräts anhand der geschätzten Hörschwelle und/oder Unbehaglichkeitsschwelle. Darüber hinaus betrifft die vorliegende Erfindung eine entsprechende Anpassvorrichtung zur Anpassung eines Hörgeräts. Ferner bezieht sich die vorliegende Erfindung auch auf ein Hörgerät mit integrierter Anpassvorrichtung.

Hörgeräte sind tragbare Hörvorrichtungen, die zur Versorgung von Schwerhörenden dienen. Um den zahlreichen individuellen Bedürfnissen entgegenzukommen, werden unterschiedliche Bauformen von Hörgeräten wie Hinter-dem-Ohr-Hörgeräte (HdO), Hörgerät mit externem Hörer (RIC: receiver in the canal) und In-dem-Ohr-Hörgeräte (IdO), z.B. auch Concha-Hörgeräte oder Kanal-Hörgeräte (ITE, CIC), bereitgestellt. Die beispielhaft aufgeführten Hörgeräte werden am Außenohr oder im Gehörgang getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.

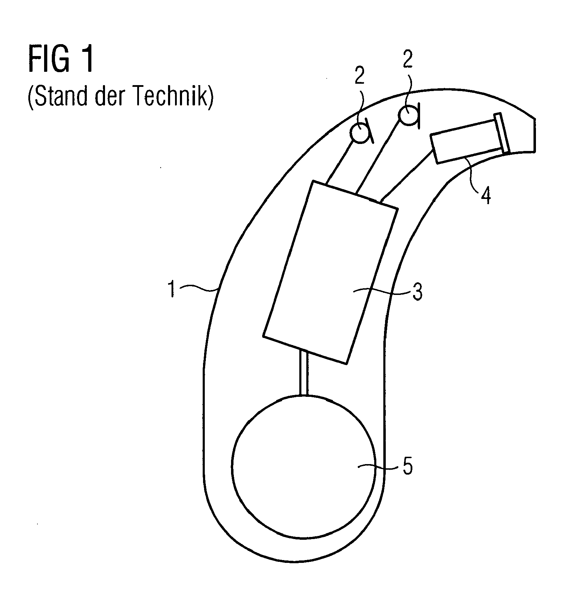

Hörgeräte besitzen prinzipiell als wesentliche Komponenten einen Eingangswandler, einen Verstärker und einen Ausgangswandler. Der Eingangswandler ist in der Regel ein Schallempfänger, z. B. ein Mikrofon, und/oder ein elektromagnetischer Empfänger, z. B. eine Induktionsspule. Der Ausgangswandler ist meist als elektroakustischer Wandler, z. B. Miniaturlautsprecher, oder als elektromechanischer Wandler, z. B. Knochenleitungshörer, realisiert. Der Verstärker ist üblicherweise in eine Signalverarbeitungseinheit integriert. Dieser prinzipielle Aufbau ist in FIG 1 am Beispiel eines Hinter-dem-Ohr-Hörgeräts dargestellt. In ein Hörgerätegehäuse 1 zum Tragen hinter dem Ohr sind ein oder mehrere Mikrofone 2 zur Aufnahme des Schalls aus der Umgebung eingebaut. Eine Signalverarbeitungseinheit 3, die ebenfalls in das Hörgerätegehäuse 1 integriert ist, verarbeitet die Mikrofonsignale und verstärkt sie. Das Ausgangssignal der Signalverarbeitungseinheit 3 wird an einen Lautsprecher bzw. Hörer 4 übertragen, der ein akustisches Signal ausgibt. Der Schall wird gegebenenfalls über einen Schallschlauch, der mit einer Otoplastik im Gehörgang fixiert ist, zum Trommelfell des Geräteträgers übertragen. Die Stromversorgung des Hörgeräts und insbesondere die der Signalverarbeitungseinheit 3 erfolgt durch eine ebenfalls ins Hörgerätegehäuse 1 integrierte Batterie 5.

Hörgeräte sind an den jeweiligen Nutzer individuell anzupassen. Die Anpassung wird in der Regel durch einen Akustiker durchgeführt, der dabei üblicherweise auf die Mitwirkung des Nutzers angewiesen ist. Gerade Kleinkinder sind hierzu wenig kooperativ. Aber auch bei Erwachsenen treten speziell dann Probleme auf, wenn bei der Bestimmung der Hörschwelle subjektive Eindrücke die Messungen verzerren, wobei eher objektive Ergebnisse erwünscht sind. Zur Vermeidung von Anpassfehlern aufgrund einer Mitwirkung des Hörgeräteträgers (beispielsweise in Form von Bestätigungen wahrgenommener Testsignale) ist man daher bestrebt, objektive Anpassverfahren einzuführen, bei denen man auf die aktive Mithilfe des Hörgeräteträgers nicht angewiesen ist.

Bei der Anpassung eines Hörgeräts ist in der Regel die Hörschwelle des Schwerhörenden zu bestimmen. Um diese objektiv zu bestimmen beziehungsweise zu schätzen, werden beispielsweise otoakustische Emissionen (OAE) des Innenohrs als Antwort auf Testsignalimpulse, die zum Trommelfell gesendet wurden, gemessen. Wie auch beim Anpassen des Hörgeräts unter Mitwirkung des Hörgeräteträgers wird beim Messen der otoakustischen Emissionen ein Audiogramm bestehend aus vielen Einzelmessungen bei mehreren Frequenzen und Amplituden erstellt.

Nachteilig an der OAE ist eine relativ lange Messdauer aufgrund der vielen Einzelmessungen. Außerdem kann es zu Messproblemen durch Überlagerungseffekte und folglich zu häufigen Wiederholungen der Einzelmessungen kommen, denn das Antwortsignal, nämlich die OAE, liegt im gleichen Frequenzbereich wie das zum Trommelfell ausgesandte Testsignal, besitzt aber eine um Größenordnungen geringere Amplitude. Des Weiteren sinkt die Zuverlässigkeit der OAE bei Schallleitungsschwerhörigkeiten.

Audiometrische Messungen auf der Basis der OAE zur Bestimmung des Hörvermögens sind beispielsweise aus der Druckschrift DE 696 25 224 T2 bekannt.

In der audiologischen Diagnostik ist außerdem die so genannte "Distortion Product" OAE (DPOAE) bekannt. Hierbei wird das Innenohr mit zwei sinusförmigen Schallreizen angeregt, deren Frequenzen in einem bestimmten Verhältnis zueinander stehen, so dass im Innenohr aufgrund von Nichtlinearitäten eine dritte Schwingung mit einer anderen Frequenz erzeugt wird. Die Bestimmung der Schwelle der Distorsionsprodukte beziehungsweise Verzerrungsprodukte otoakustischer Emissionen (DPOAE) ist eine Methode, die unabhängig vom Pegel der Emission arbeitet. Daher ergeben sich Vorteile bei Schallleitungsschwerhörigkeiten (vergleiche Peter Kummer und Ulrich Eysholdt: "Der Einfluss einer Schallleitungsschwerhörigkeit auf die DPOAE-Schwelle"; 20. Wissenschaftliche Jahrestagung der Deutschen Gesellschaft für Phoniatrie und Pädaudiologie; 12. bis 14. September 2003, Rostock). Auch J. A. Oswald et al. nutzen für die audiologische Diagnostik die Tatsache, dass die audiometrische Hörschwelle und die DPOAE-Schwelle eng zusammenhängen, wenn zur Auslösung der DPOAE eine so genannte "Pegelschere" verwendet wird und die Emissionen mit hörschwellennahen Reizen ausgelöst werden (vergleiche J. A. Oswald et al.: "Messung der DPOAE im erweiterten Reizpegelbereich - Ein Vergleich mit Basilarmembranschnelle und Lautheit"; DFG Ja 597/6).

Die Aufgabe der vorliegenden Erfindung besteht darin, die Anpassung eines Hörgeräts zu verbessern und eine entsprechende Anpassvorrichtung beziehungsweise ein entsprechendes Anpassverfahren vorzuschlagen.

Erfindungsgemäß wird diese Aufgabe gelöst durch ein Verfahren zur Anpassung eines Hörgeräts durch Messen von otoakustischen Emissionen, Schätzen einer Hörschwelle und/oder einer Unbehaglichkeitsschwelle aus den Messungen und Einstellen des Hörgeräts anhand der geschätzten Hörschwelle und/oder Unbehaglichkeitsschwelle, wobei das Gehör des Hörgerätsträgers gleichzeitig mit mindestens zwei Testsignalen unterschiedlicher Frequenz stimuliert wird, und wobei beim Messen der otoakustischen Emissionen Verzerrungsprodukte festgestellt und zum Schätzen der Hörschwelle und/oder Unbehaglichkeitsschwelle herangezogen werden. Dabei kann unter dem "Schätzen einer Hörschwelle beziehungsweise Unbehaglichkeitsschwelle" auch das Schätzen eines oder mehrerer Werte, aus denen die Hörschwelle beziehungsweise Unbehaglichkeitsschwelle berechnet werden kann, verstanden werden.

Darüber hinaus wird erfindungsgemäß bereitgestellt eine Anpassvorrichtung zur Anpassung eines Hörgeräts mit einer Messeinrichtung zum Messen otoakustischer Emissionen, einer Recheneinrichtung zum Schätzen einer Hörschwelle und/oder Unbehaglichkeitsschwelle aus den Messungen und zum Erstellen mindestens eines Parameterwerts aus der Schätzung sowie einer Übertragungseinrichtung zum Übertragen des mindestens einen Parameterwerts an eine Steuereinheit des Hörgeräts, wobei von der Messeinrichtung das Gehör des Hörgeräteträgers gleichzeitig mit mindestens zwei Testsignalen unterschiedlicher Frequenz stimulierbar ist, von der Messeinrichtung Verzerrungsprodukte der otoakustischen Emissionen feststellbar sind und die Recheneinrichtung die Verzerrungsprodukte zum Schätzen der Hörschwelle und/oder Unbehaglichkeitsschwelle heranzieht.

Der erfindungsgemäße Einsatz der DPOAE zur Hörgeräteanpassung und insbesondere zur Selbstanpassung führt zu einer deutlichen Beschleunigung des Anpassverfahrens, da die Messergebnisse bezüglich der otoakustischen Emissionen zuverlässiger sind und daher weniger Wiederholungen von Einzelmessungen notwendig sind. Außerdem treten weniger Probleme bei Schallleitungsschwerhörigkeiten auf.

Vorzugsweise werden zum Stimulieren zwei Testsignale eingesetzt, die der Pegelschere L1 = 0,4 L2 + 39 dB genügen, worin L1 und L2 die Pegel der beiden Testsignale darstellen. Dabei ist es auch günstig, wenn zum Stimulieren zwei Testsignale mit den Frequenzen f1 und f2 verwendet werden, deren Frequenzverhältnis f2/f1 = 1,2 ist beziehungsweise im Bereich 1,1 ≤ f2/f1 ≤ 1,3 liegt. Derartige Testsignale führen zu deutlich ausgeprägten Verzerrungsprodukten. Weichen die Testsignale von den obigen Bedingungen ab, so sind entsprechend niedrigere Emissionspegel der Verzerrungsprodukte zu erwarten.

Im Rahmen der Anpassung kann beispielsweise die Zielverstärkung des Hörgeräts anhand der geschätzten Hörschwelle und/oder Unbehaglichkeitsschwelle eingestellt werden. Alternativ oder zusätzlich können aber auch andere Parameter beziehungsweise Algorithmen wie beispielsweise zur Kompression oder Störgeräuschunterdrückung angepasst werden.

Besonders bevorzugt ist, wenn das Hörgerät in der Lage ist, eine Selbstanpassung durchzuführen. Hierzu ist die oben beschriebene Anpassvorrichtung in das Hörgerät integriert, wobei ein Hörer sowohl zur Ausgabe eines verstärkten Nutzsignals als auch zur Ausgabe der Testsignale der Messeinrichtung dient, und wobei im eingesetzten Zustand des Hörgeräts ein Mikrofon der ins Hörgerät integrierten Messeinrichtung im Ohrkanal angeordnet ist. Dadurch ist es nicht mehr notwendig, dass der Hörgeräteträger zum Anpassen einen Akustiker aufsucht. Ein besonderer Vorteil der Selbstanpassung liegt aber auch darin, dass gerade bei Kleinkindern, bei denen sich das Gehör in verhältnismäßig kurzen Zeiträumen verändert, in kurzen Zeitabständen beziehungsweise dynamisch ein Anpassprozess durchgeführt werden kann.

Die vorliegende Erfindung wird anhand der beigefügten Zeichnungen näher erläutert, in denen zeigen:
- FIG 1: den prinzipiellen Aufbau eines Hörgeräts gemäß dem Stand der Technik;
- FIG 2: den prinzipiellen Aufbau einer Anpassvorrichtung zum Anpassen eines Hörgeräts und
- FIG 3: ein IdO-Hörgerät mit integrierter Anpassvorrichtung.

Die nachfolgend näher geschilderten Ausführungsbeispiele stellen bevorzugte Ausführungsformen der vorliegenden Erfindung dar.

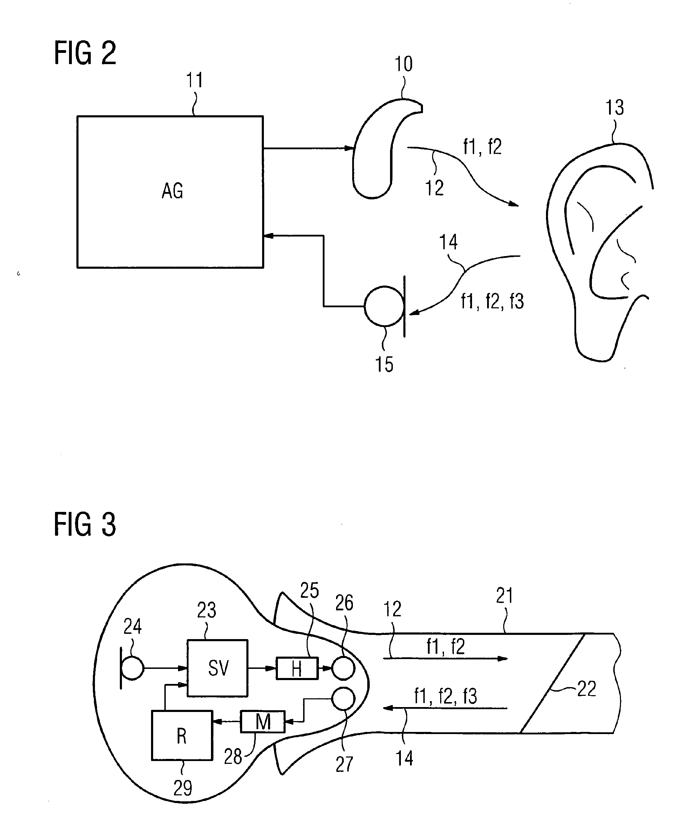

Entsprechend dem Beispiel von FIG 2 soll ein HdO-Hörgerät 10 angepasst werden. Es wird hierzu an ein Anpassgerät 11 angeschlossen. Das Anpassgerät 11 steuert das Hörgerät 10 so an, dass dessen Hörer Testsignale 12 in den Gehörgang des Ohrs 13 abgibt. Während der Anpassung wird das Hörgerät 10 am Ohr 13 getragen und die Testsignale 12 werden beispielsweise mittels eines Schallschlauchs in den Gehörgang geleitet. Alternativ kann es sich bei dem Hörgerät auch um ein RIC-Gerät handeln, so dass die akustischen Testsignale direkt mithilfe eines externen Hörers im Gehörgang erzeugt werden.

Akustische Emissionen 14, die vom Gehörgang abgestrahlt werden, werden durch ein Sondenmikrofon 15, das ebenfalls in den Gehörgang geführt ist, aufgenommen. Das Sondenmikrofon 15 leitet die entsprechenden elektrischen Signale zur Auswertung in das Anpassgerät 11. Dort werden aus den Mikrofonsignalen Anpassparameter berechnet, die dann wiederum an das Hörgerät 10 übertragen werden.

Für die Anpassung wird die "Distortion Product" OAE (DPOAE) verwendet. Hierbei werden Nichtlinearitätseffekte des Innenohrs genutzt. Speziell werden Sinustöne zweier Frequenzen f1 und f2 (Testsignale) vom Hörgerätehörer gleichzeitig erzeugt und zum Trommelfell gesandt. Die Nichtlinearitätseffekte in der Cochlea führen zur Ausbildung einer weiteren Frequenz f3 als Verzerrungsprodukt ("Distortion Product") der beiden anregenden Frequenzen f1 und f2. Diese neue Frequenz f3 gelangt über das Mittelohr und das Trommelfell in den Ohrkanal und kann dort aufgrund der unterschiedlichen Frequenz zu den Testsignalen f1 und f2 mittels eines Filters selektiv und sicher bestimmt werden.

In FIG 3 ist eine zweite Ausführungsform der vorliegenden Erfindung dargestellt. Es handelt sich hier um ein IdO-Hörgerät 20, das in einen Gehörgang 21 eingesetzt wird. Dieser ist zum Mittelohr durch das Trommelfell 22 getrennt. Das IdO-Hörgerät besitzt eine Signalverarbeitungseinheit 23, die üblicherweise von einem Mikrofon 24 Mikrofonsignale aufnimmt und verstärkt. Die verstärkten Signale werden an einen Hörer 25 weitergeführt, der entsprechende akustische Ausgangssignale erzeugt. Diese treten an einer Schallaustrittsöffnung 26 aus dem IdO-Hörgerät 20 aus und gelangen direkt durch den Gehörgang 21 zum Trommelfell 22.

Für das erfindungsgemäße objektive Anpassverfahren wird nun diese übliche Hörgerätestruktur genutzt um Testsignale zu generieren. Hierbei erzeugt die Signalverarbeitungseinheit 23 entsprechende elektrische Signale beziehungsweise stellt sie bereit. Der Hörer 25 wandelt diese in akustische Testsignalimpulse 12 der Frequenzen f1 und f2. Nichtlinearitäten des Trommelfells, Mittelohrs und Innenohrs führen zu einer Antwort beziehungsweise zu einem Echo 14 mit den drei Frequenzen f1, f2 und f3. Diese Antwort wird von dem IdO-Hörgerät 20 durch eine Mikrofonöffnung 27 aufgenommen und in einer Messeinrichtung 28 erfasst. Die Messergebnisse werden einer Recheneinrichtung 29 zugeführt, welche aus den DPOAE-Messergebnissen eine Hörschwelle und/oder Unbehaglichkeitsschwelle beziehungsweise Parameter für die Signalverarbeitungseinrichtung 23 berechnet. Anhand der Hörschwelle beziehungsweise der errechneten Parameter wird die Signalsverarbeitungseinheit 23 an den Hörgeräteträger angepasst. Es wird hier also ein für die üblicherweise klinische Diagnose genutztes Verfahren, die DPOAE, zur Analyse des Hörvermögens mithilfe eines Hörgeräts genutzt. Dabei erfolgt nicht nur die gesamte Analyse, sondern auch die Schallerzeugung und die Schallaufnahme der Testsignale beziehungsweise Testsignalantworten durch das Hörgerät selbst. Es erfolgt somit eine objektive Selbstanpassung des Hörgeräts ohne aktive Mithilfe des Hörgerätsträgers.

## Patentansprüche

1. Verfahren zur Anpassung eines Hörgeräts (10, 20) durch
- Messen von otoakustischen Emissionen (14),
- Schätzen einer Hörschwelle und/oder einer Unbehaglichkeitsschwelle aus den Messungen und
- Einstellen des Hörgeräts (10, 20) anhand der geschätzten Hörschwelle und/oder Unbehaglichkeitsschwelle,
**gekennzeichnet durch**
- Stimulieren des Gehörs des Hörgerätsträgers gleichzeitig mit mindestens zwei Testsignalen (12) unterschiedlicher Frequenz (f1, f2), wobei
- beim Messen der otoakustischen Emissionen (14) Verzerrungsprodukte (f3) festgestellt und zum Schätzen der Hörschwelle und/oder Unbehaglichkeitsschwelle herangezogen werden.

2. Verfahren nach Anspruch 1, wobei zum Stimulieren zwei Testsignale (12) eingesetzt werden, die der Pegelschere L1 = 0,4 L2 + 39 dB genügen, worin L1 und L2 die Pegel der beiden Testsignale darstellen.

3. Verfahren nach Anspruch 1 oder 2, wobei zum Stimulieren zwei Testsignale (12) mit den Frequenzen f1 und f2 verwendet werden, deren Frequenzverhältnis im Bereich 1,1 ≤ f2/f1 ≤ 1,3 liegt und insbesondere f2/f1 = 1,2 ist.

4. Verfahren nach einem der vorhergehenden Ansprüche, wobei eine Zielverstärkung des Hörgeräts anhand der geschätzten Hörschwelle und/oder Unbehaglichkeitsschwelle eingestellt wird.

5. Anpassvorrichtung zur Anpassung eines Hörgeräts (10, 20) mit
- einer Messeinrichtung (28) zum Messen otoakustischer Emissionen,
- einer Recheneinrichtung (29) zum Schätzen einer Hörschwelle und/oder Unbehaglichkeitsschwelle aus den Messungen und zum Erstellen mindestens eines Parameterwerts aus der Schätzung sowie
- einer Übertragungseinrichtung zum Übertragen des mindestens einen Parameterwerts an eine Steuereinheit (23) des Hörgeräts,
**dadurch gekennzeichnet, dass**
- von der Messeinrichtung (28) das Gehör des Hörgeräteträgers gleichzeitig mit mindestens zwei Testsignalen (12) unterschiedlicher Frequenz stimulierbar ist,
- von der Messeinrichtung (28) Verzerrungsprodukte (f3) der otoakustischen Emissionen (14) feststellbar sind und
- die Recheneinrichtung (29) die Verzerrungsprodukte (f3) zum Schätzen der Hörschwelle und/oder Unbehaglichkeitsschwelle heranzieht.

6. Anpassvorrichtung nach Anspruch 5, wobei die Messeinrichtung (28) zwei Testsignale (12) ausgibt, die der Pegelschere L1 = 0,4 L2 + 39 dB genügen, wobei L1 und L2 die Pegel der beiden Testsignale darstellen.

7. Anpassvorrichtung nach einem der Ansprüche 5 oder 6, wobei die Messeinrichtung (28) zwei Testsignale (12) mit den Frequenzen f1 und f2 ausgibt, deren Frequenzverhältnis im Bereich 1,1 ≤ f2/f1 ≤ 1,3 liegt und insbesondere f2/f1 = 1,2 ist.

8. Hörgerät (10, 20), in das eine Anpassvorrichtung nach einem der Ansprüche 5 bis 7 integriert ist, wobei ein Hörer (25) sowohl zur Ausgabe eines verstärkten Nutzsignals als auch zur Ausgabe der Testsignale (12) der Messeinrichtung (28) dient, und wobei im eingesetzten Zustand des Hörgeräts (10, 20) ein Mikrofon der Messeinrichtung (28) im Ohrkanal (21) angeordnet ist.
